# EUROPEAN PATENT SPECIFICATION

(11) **EP 4 293 211 B1**
(45) Date of publication and mention of the grant of the patent: **14.08.2024**
(21) Application number: 23178691.4
(22) Date of filing: 12.06.2023
(51) Int. Cl.: F02B 77/08, F01L 1/14, F16C 33/04, G01M 15/04

(54) **A METHOD OF LUBRICATING A DLC COATED TAPPET PIN**
VERFAHREN ZUM SCHMIEREN EINES DLC-BESCHICHTETEN STÖSSELSTIFTES
PROCÉDÉ DE LUBRIFICATION D'UNE TIGE DE POUSSOIR REVÊTUE DE DLC

(30) Priority: 13.06.2022 NL 2032156
(43) Date of publication of application: 20.12.2023
(73) Proprietor: DAF Trucks N.V., 5643 TW Eindhoven (NL)
(72) Inventor: LIU, Xin, 5643 TW Eindhoven (NL); VAN DROGEN, Mark, 5643 TW Eindhoven (NL); WIJN, Alexander Franciscus Maria, 5643 TW Eindhoven (NL)
(74) Representative: V.O.

(56) References cited:
- KR-A- 20190 024 846
- US-A1- 2005 098 134
- US-B1- 6 844 068
- US-B2- 6 886 521

## Description

### BACKGROUND OF THE INVENTION

Tappets are known moving parts in a combustion engine that is operated with a camshaft principle. The tappet comprises a static tappet pin, around which a tappet roller is rotating. The contact between tappet pin and roller is a sliding contact within the diameter of the roller. The contact between the cam and roller is outside of the roller and is designed as a rolling contact. The tappet roller, as a cam follower, translates rotary movement of the cam shaft into a reciprocating movement in order to control engine valves or other movable parts of the combustion engine.

Advances in the construction of these tappet pins are the use of DLC (diamond like carbon) coatings, that provide the tappet pin surface with a very hard surface coating for increasing wear resistance, last to endure many revolutions, that the engine will encounter in an economic lifetime of about more than 1.6 million kilometres (1 million miles) work.

See for example US6844068, US2005098134, US6886521, and KR20190024846. Recently, it was found that these coatings are vulnerable to certain types of lubricant oil, while these have the function to minimize the friction in the sliding contact between tappet roller and (static) tappet pin of the cam follower, can also weaken the coating. See Ohara et al. "Analysis of Wear Track on DLC Coatings after with MoDTC-Containing Lubricants", Tribology online vol 12, No 3(2017)110-116. Under highly concentrated local contact pressure, the DLC coating appears to be gradually weakened until it no longer functions as a smooth frictionless surface, and the tappet is in need of replacement, adding to maintenance costs. Unfortunately, while such Mo compounds are in principle detectable through chemical analysis or through information supplied by the manufacturer, it is in practice difficult to estimate the adverse effect of using certain types of oil, due to varying amounts of additives which adds to the difficulty in predicting economic lifetime. To this end a method of selecting an engine oil for a DLC coated tappet pin is presented that overcomes these problems in an efficient way, since an efficient, reproducible and fast selection test is provided.

### SUMMARY OF THE INVENTION

The invention concerns a method of lubricating a DLC coated tappet pin in a combustion engine, comprising:
providing an oil cup on a holder substrate, said cup having a static member mounted therein immersed in an oil under selection;
mounting the tappet pin to a distal end part of a reciprocating actuator; applying a downward force to establish a contact pressure between the tappet pin and the static member, while actuating the tappet pin, by the actuator, in reciprocating motion over the static pin, the static member oriented transverse to the tappet pin;
measuring a friction force signal of the oil cup relative to the holder substrate in a predetermined time frame; and
rejecting the oil under selection for lubricating the tappet pin in the engine, when a friction signal drops below a threshold level in the predetermined time frame; or else selecting the oil under selection to lubricate the tappet pin in the engine.

The invention will further be elucidated by description of some specific embodiments thereof, making reference to the attached drawings. The detailed description provides examples of possible implementations of the invention, but is not to be regarded as describing the only embodiments falling under the scope. The scope of the invention is defined in the claims, and the description is to be regarded as illustrative without being restrictive on the invention.

### BRIEF DESCRIPTION OF FIGURES

Figure 1 shows an exemplary oil selection test according to the invention.
Figure 2 shows a basic setup for a providing the oil selection test;
Figure 3 (A and B) shows the schematic setup for carrying out the oil selection test according to the invention.

### DETAILED DESCRIPTION

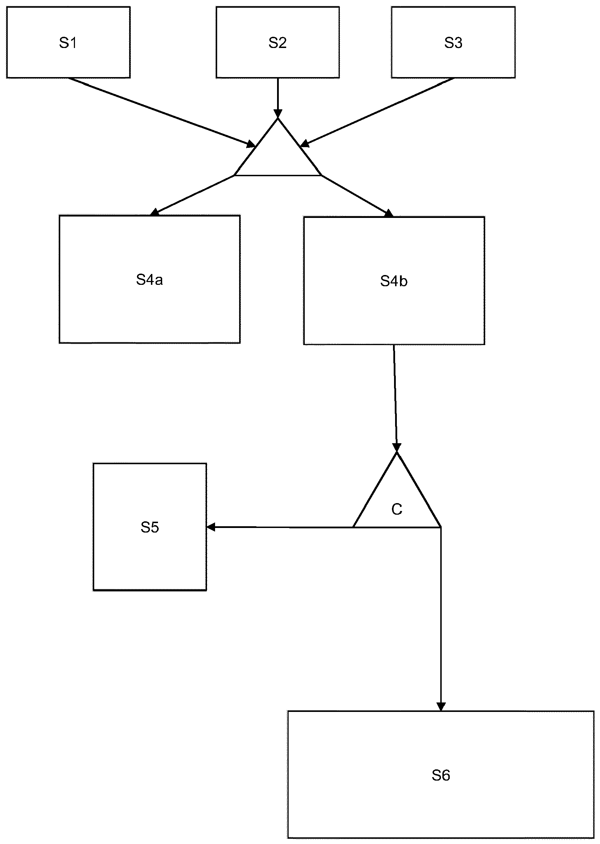

Figure 1 shows an exemplary lubricating method according to the invention to lubricate a DLC coated tappet pin in a combustion engine. In a first step S1, an oil cup is provided on a holder substrate, i.e. a semi open container of any particular shape e.g. a rectangular open metal box or the like. In a further step S2 a static member is mounted in the container, and immersed in the oil under selection. The tappet pin, in a further step S3 is mounted fixed to a distal end part of a reciprocating actuator. Steps S1, S2 and S3 can be considered as preparatory steps to be applied without any particular order. Subsequently a downward pressure is applied in step S4a between the tappet pin and the static member, while, at the same time the tappet pin is actuated, by the actuator, in reciprocating motion over the static member, oriented transverse to the tappet pin. At the same time, while moving the tapped pin over the static pin under downward pressure, a friction force signal is measured (S4b) of the oil cup relative to the holder substrate in a predetermined time frame. The oil under selection for lubricating the tappet pin in the engine is rejected (S5) in a comparison step C when a friction signal drops below a threshold level in the predetermined time frame. While the signal drop may depend on specific setup parameters of the test setup and can preferably be determined by calibration, by way of example it can be defined to exceed a threshold if the friction signal drops with a percentage of at least 10%, e.g. 20%. If such a signal drop does not occur the oil under selection may be selected to lubricate the bearing contact between tappet pin and tappet roller in the engine (S6). Since the intended use of the oil under test is to reduce friction it is noted that there is a counter intuitive aspect, that the oil under selection is not selected when the friction force drops, under the influence of friction reducing additives. Without being limited to the theory, it is surmised that the friction force drop occurs due to the instability of the DLC coating, that fails due to the occurrence of certain friction reducing additives.

This signal drop, when measured, can be used to identify a suitable engine oil for lubricating the bearing surface between tappet pin and tappet roller. Indeed it can be seen, from further inspection, that in such conditions the coating appears to be damaged, which would result in a reduction of life span of the tappet pin when lubricated by the selected oil under test.

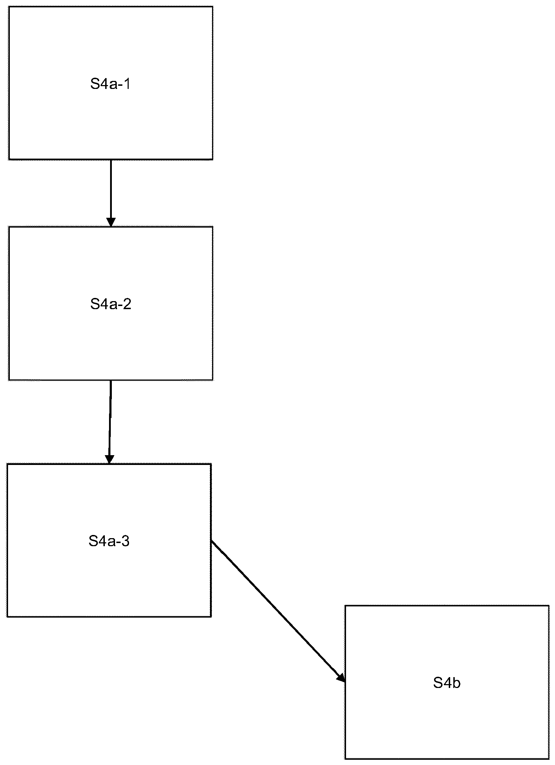

Figure 2 provides a further detailed embodiment of the measurement steps S4a, S4b. To optimally mimic the conditions wherein the lubrication of the tappet takes place in the engine, preferably in a first heating step S4a-1 a contact pressure is applied of about 1200 MPa e.g. exceeding 1000 MPa, while the oil is heated to about 90 °Celsius, or any suitable value e.g. ranging from 80-95 °C. In this phase S4a-1, the exerted contact pressure is moderate, and can, for a given geometry be provided by an actuation force in the order of 10 Newton. In a subsequent 'loading' phase, when the oil is suitably heated the contact pressure is gradually increased, while the oil is kept at an elevated temperature. E.g. loading force will increase from about 10 to 100 Newton in loading step S4a-2 with a contact pressure increasing to 2500 MPa or more in a time frame of about 20 -40 minutes. These steps are carried out with the tappet pin in sliding movement actuated over the static member. In a subsequent friction measurement step S4a-3 a friction signal is measured in a time frame ranging between 4 and 6 hours.

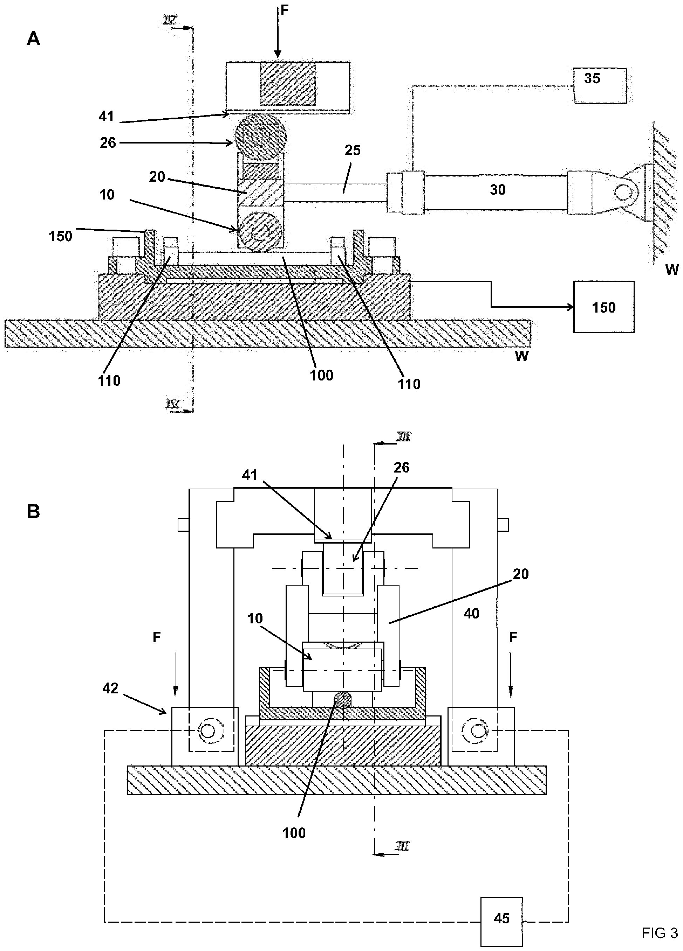

Figure 3 shows a schematic setup for carrying out the disclosed method, where 3A and 3B are side views and frontal views of the setup, seen along lines III and IV respectively. A tappet pin 10 is provided mounted in fixed contact to a distal end part 20 of a piston 25 actuated by an actuator 30 that is in connection with the fixed world W. Downward pressure F is exerted to tappet pin 10 which, is initially in the order of 1000 MPa, but during signal measurement may exceed 2500 MPa. To this end a yoke 40 is provided having a runner surface 41. The runner surface 41, by means of actuator 42 exerts a downward force controlled by a controller 45 to guiding roller 26 which can rotate along he runner surface 41. Guiding roller 26, is provided opposite tappet pin 10, and equally mounted to distal end part 20. For example this end part 20 can be realized by an 'H' mounting piece fixedly connected to piston 25, having upper and lower extending arms respectively, between which the guiding roller 26 and tappet pin 10 are mounted, guiding roller 26 in a roll bearing; tappet pin 10 in fixed mounted contact. Thus, by exerting downward pressure via running surface 41, tappet pin is pressed downward to a static member 100. Member 100, e.g. a stainless steel pin can be secured by clamps 110 in cup container 150, preferably to provide a line contact between the contacting interface of two cylinders. Container 150 is suitable to receive member 100 and fill it with lubrication oil under test, so that the contact surface between pin 100 and tappet pin 10 is immersed. As can be seen from Figures 3A and 3B, the orientation of static pin 100 is so that, when the tappet pin 10 is actuated in reciprocating motion over the static pin 100, the static pin 100 is oriented transverse to the tappet pin 10 with respective longitudinal axes. Container 150 may be mounted on a flexible mounting table, that is connected to the fixed world W via a friction sensor 150, able to measure the friction force exerted on the member 100, when tappet pin 10 is moved over the static member 100 in sliding contact, with a reciprocating motion e.g. ranging between 5 and 15 Hz, preferably around 10 Hz. A typical stroke length can range between 5 and 15 mm.

To suitably function as a counter surface for the DLC coated tappet pin 10, static member is of a hardened material, but does not have a DLC coating. Preferably it is a steel pin, e.g. a 6 mm Ø × 50 mm length DIN 6325 Steel with a hardness of 60 ± 2 HRC.

It will be clear to the skilled person that the invention is not limited to any embodiment herein described and that modifications are possible which may be considered within the scope of the appended claims. Also kinematic inversions are considered inherently disclosed and can be within the scope of the invention. In the claims, any reference signs shall not be construed as limiting the claim. The terms 'comprising' and 'including' when used in this description or the appended claims should not be construed in an exclusive or exhaustive sense but rather in an inclusive sense. Thus expression as 'including' or 'comprising' as used herein does not exclude the presence of other elements, additional structure or additional acts or steps in addition to those listed. Furthermore, the words 'a' and 'an' shall not be construed as limited to 'only one', but instead are used to mean `at least one', and do not exclude a plurality. Features that are not specifically or explicitly described or claimed may additionally be included in the structure of the invention without departing from its scope. Expressions such as: "means for ..." should be read as: "component configured for ..." or "member constructed to ..." and should be construed to include equivalents for the structures disclosed. The use of expressions like: "critical", "preferred", "especially preferred" etc. is not intended to limit the invention. To the extent that structure, material, or acts are considered to be essential they are inexpressively indicated as such. Additions, deletions, and modifications within the purview of the skilled person may generally be made without departing from the scope of the invention, as determined by the claims.

## Claims

1. A method of lubricating a DLC coated tappet pin (10) in a combustion engine, comprising
- providing (S1) an oil cup (150) on a holder substrate, said cup having a static member (100) mounted (S2) therein immersed in an oil under selection;
- mounting (S3) the tappet pin (10) to a distal end part (20) of a reciprocating actuator (30);
- applying (S4a) a downward force (F) to establish a contact pressure between the tappet pin (10) and the static member (100), while actuating the tappet pin (10), by the actuator, in reciprocating motion over the static member (100), the static member (100) oriented transverse to the tappet pin (10);
- measuring (S4b) a friction force signal of the oil cup (150) relative to the holder substrate in a predetermined time frame; **characterized by**
- rejecting (S5) the oil under selection for lubricating the tappet pin (10) in the engine, when a friction signal drops below a threshold level in the predetermined time frame; or else selecting (S6) the oil under selection to lubricate the tappet pin (10) in the engine.

2. The selection method according to claim 1, wherein the contact pressure exceeds 2500 MPa.

3. The selection method according to any preceding claim, wherein the friction signal drops with a percentage of at least 20%.

4. The selection method according to any preceding claim, wherein the static member (100) has a hardness of 60 HRC.

5. The selection method according to any preceding claim, wherein the downward pressure (F) is applied with the oil heated to a temperature exceeding 80° Celsius.

6. The selection method according to any preceding claim, wherein the downward force (F) is applied with a reciprocating motion ranging between 5 and 15 Hz.

7. The selection method according to any preceding claim, wherein the downward force (F) is applied with a stroke length ranging between 5 and 15 mm.

8. The selection method according to any preceding claim, wherein the downward force (F) is applied:
- in a first heating step (S4a-1) with a contact pressure between tappet pin (10) and static member (100) exceeding 1000 MPa; followed by
- a loading step (S4a-2) with a contact pressure increasing to 2500 MPa in a time frame of more than 20 minutes;
- a friction measurement step (S4a-3) in a time frame ranging between 4 and 6 hours.

## Patentansprüche

1. Verfahren zum Schmieren eines DLC-beschichteten Stößelstifts (10) in einem Verbrennungsmotor, umfassend
- Bereitstellen (S1) eines Ölbechers (150) auf einem Trägersubstrat, wobei der Becher ein statisches Element (100) aufweist, das darin angebracht ist (S2) und in ein ausgewähltes Öl eingetaucht ist;
- Anbringen (S3) des Stößelstifts (10) an einem distalen Endteil (20) einer hin- und hergehenden Betätigungsvorrichtung (30);
- Aufbringen (S4a) einer nach unten gerichteten Kraft (F), um einen Anpressdruck zwischen dem Stößelstift (10) und dem statischen Element (100) herzustellen, während der Stößelstift (10) durch die Betätigungsvorrichtung in einer hin- und hergehenden Bewegung über das statische Element (100) bewegt wird, wobei das statische Element (100) quer zum Stößelstift (10) ausgerichtet ist;
- Messen (S4b) eines Reibkraftsignals des Ölbechers (150) relativ zu dem Trägersubstrat in einem vorbestimmten Zeitrahmen; **gekennzeichnet durch**
- Zurückweisen (S5) des ausgewählten Öls zum Schmieren des Stößelstifts (10) in dem Motor, wenn ein Reibungssignal in dem vorbestimmten Zeitrahmen unter einen Schwellenwert fällt; oder andernfalls Auswählen (S6) des ausgewählten Öls zum Schmieren des Stößelstifts (10) in dem Motor.

2. Auswahlverfahren nach Anspruch 1, wobei der Anpressdruck 2500 MPa übersteigt.

3. Auswahlverfahren nach einem der vorstehenden Ansprüche, wobei das Reibungssignal mit einem Prozentsatz von mindestens 20 % abfällt.

4. Auswahlverfahren nach einem der vorstehenden Ansprüche, wobei das statische Element (100) eine Härte von 60 HRC aufweist.

5. Auswahlverfahren nach einem der vorstehenden Ansprüche, wobei der Abwärtsdruck (F) ausgeübt wird, während das Öl auf eine Temperatur von über 80° Celsius erhitzt ist.

6. Auswahlverfahren nach einem der vorstehenden Ansprüche, wobei die nach unten gerichtete Kraft (F) mit einer hin- und hergehenden Bewegung zwischen 5 und 15 Hz aufgebracht wird.

7. Auswahlverfahren nach einem der vorstehenden Ansprüche, wobei die Abwärtskraft (F) mit einer Hublänge zwischen 5 und 15 mm aufgebracht wird.

8. Auswahlverfahren nach einem der vorstehenden Ansprüche, wobei die nach unten gerichtete Kraft (F) aufgebracht wird:
- in einem ersten Erwärmungsschritt (S4a-1) mit einem Anpressdruck zwischen Stößel (10) und statischem Element (100), der 1000 MPa übersteigt; gefolgt von
- einem Belastungsschritt (S4a-2) mit einem auf 2500 MPa ansteigenden Anpressdruck in einem Zeitrahmen von mehr als 20 Minuten;
- einen Schritt zur Messung der Reibung (S4a-3) in einem Zeitrahmen zwischen 4 und 6 Stunden.

## Revendications

1. Procédé de lubrification d'une tige de poussoir revêtue de DLC (10) dans un moteur à combustion, comprenant les étapes consistant à
- fournir (S1) une coupelle d'huile (150) sur un substrat de support, ladite coupelle ayant un élément statique (100) monté en son sein (S2) immergé dans une huile sous sélection ;
- monter (S3) la tige de poussoir (10) sur une partie d'extrémité distale (20) d'un actionneur à mouvement alternatif (30) ;
- appliquer (S4a) une force vers le bas (F) pour établir une pression de contact entre la tige de poussoir (10) et l'élément statique (100), tout en actionnant la tige de poussoir (10), par l'intermédiaire de l'actionneur, dans un mouvement alternatif sur l'élément statique (100), l'élément statique (100) étant orienté transversalement à la tige de poussoir (10) ;
- mesurer (S4b) un signal de force de frottement de la coupelle d'huile (150) par rapport au substrat de support dans un laps de temps prédéterminé ; **caractérisé par** les étapes consistant à
- rejeter (S5) l'huile sous sélection pour lubrifier la tige de poussoir (10) dans le moteur, lorsqu'un signal de frottement tombe en dessous d'un niveau de seuil dans le laps de temps prédéterminé ; ou bien sélectionner (S6) l'huile sous sélection pour lubrifier la tige de poussoir (10) dans le moteur.

2. Procédé de sélection selon la revendication 1, dans lequel la pression de contact dépasse 2500 MPa.

3. Procédé de sélection selon l'une quelconque des revendications précédentes, dans lequel le signal de frottement chute avec un pourcentage d'au moins 20 %.

4. Procédé de sélection selon l'une quelconque des revendications précédentes, dans lequel l'élément statique (100) présente une dureté de 60 HRC.

5. Procédé de sélection selon l'une quelconque des revendications précédentes, dans lequel la pression vers le bas (F) est appliquée avec l'huile chauffée à une température dépassant 80°Celsius.

6. Procédé de sélection selon l'une quelconque des revendications précédentes, dans lequel la force vers le bas (F) est appliquée avec un mouvement alternatif compris entre 5 et 15 Hz.

7. Procédé de sélection selon l'une quelconque des revendications précédentes, dans lequel la force vers le bas (F) est appliquée avec une longueur de course comprise entre 5 et 15 mm.

8. Procédé de sélection selon l'une quelconque des revendications précédentes, dans lequel la force descendante (F) est appliquée :
- dans une première étape de chauffage (S4a-1) avec une pression de contact entre la tige de poussoir (10) et l'élément statique (100) supérieure à 1000 MPa ; suivie par
- une étape de chargement (S4a-2) avec une pression de contact allant jusqu'à 2500 MPa dans un laps de temps de plus de 20 minutes ;
- une étape de mesure de frottement (S4a-3) dans un laps de temps compris entre 4 et 6 heures.
